# EUROPEAN PATENT SPECIFICATION

(11) **EP 3 498 114 B1**
(45) Date of publication and mention of the grant of the patent: **21.07.2021**
(21) Application number: 17208252.1
(22) Date of filing: 18.12.2017
(51) Int. Cl.: A24F 40/485, A24F 40/10, A24F 15/015

(54) **ELECTRONIC SMOKING DEVICE WITH LIQUID DUCT**
ELEKTRONISCHE RAUCHVORRICHTUNG MIT FLÜSSIGKEITSKANAL
DISPOSITIF À FUMER ÉLECTRONIQUE POURVU D'UN CONDUIT DE LIQUIDE

(43) Date of publication of application: 19.06.2019
(73) Proprietor: Fontem Holdings 1 B.V., 1083 HN Amsterdam (NL)
(72) Inventor: Biel, Stefan, 22761 Hamburg (DE); Jones, David, Halton Brook, Runcorn, Cheshire, WA7 2SS (GB)
(74) Representative: Gulde & Partner

(56) References cited:
- EP-A2- 3 143 884
- WO-A1-2015/027436
- CN-U- 206 603 250
- DE-U1-202015 001 439
- US-A1- 2016 128 384
- US-A1- 2017 105 449

## Description

### FIELD OF INVENTION

The present invention relates generally to electronic smoking devices and in particular electronic cigarettes. The present invention in particular relates to electronic smoking devices having a refillable liquid reservoir.

### BACKGROUND OF THE INVENTION

An electronic smoking device, such as an electronic cigarette (e-cigarette), typically has a housing accommodating an electric power source (e.g. a single use or rechargeable battery, electrical plug, or other power source), and an electrically operable atomizer. The atomizer vaporizes or atomizes liquid supplied from a reservoir and provides vaporized or atomized liquid as an aerosol. Control electronics control the activation of the atomizer. In some electronic cigarettes, an airflow sensor is provided within the electronic smoking device, which detects a user puffing on the device (e.g., by sensing an under-pressure or an air flow pattern through the device). The airflow sensor indicates or signals the puff to the control electronics to power up the device and generate vapor. In other e-cigarettes, a switch is used to power up the e-cigarette to generate a puff of vapor.

An electronic smoking device can be adapted to allow refilling a liquid reservoir.

EP 3143884 A2 discloses an electronic smoking device comprising a power supply portion and an atomizer/liquid reservoir portion comprising a refillable liquid reservoir adapted for storing a liquid, and an atomizer operable when connected to the power supply to atomize liquid stored in the liquid reservoir. The atomizer/liquid reservoir portion comprises a liquid filling channel and an air outlet channel at least partially separate from the liquid filling channel.

### SUMMARY OF THE INVENTION

In accordance with one aspect of the present invention, there is provided an electronic smoking device that comprises a power supply portion comprising a power supply, and an atomizer/liquid reservoir portion. The atomizer/liquid reservoir portion comprises a refillable liquid reservoir adapted for storing liquid, an atomizer operable when connected to the power supply to atomize liquid stored in the liquid reservoir, and a central passage that is adapted to conduct atomized liquid, the central passage extending through the refillable liquid reservoir. The atomizer/liquid reservoir portion comprises a docking valve for docking a source of liquid to be stored in the liquid reservoir, a liquid duct that is adapted to receive liquid from the docking valve. The liquid duct opens into the refillable liquid reservoir at a distance to the docking valve, and a valve for releasing gas from the refillable liquid reservoir when refilling the refillable liquid reservoir, the valve comprising a flexible sealing element and an essentially rigid counter sealing element formed by an outer side wall section of the central passage.

The characteristics, features and advantages of this invention and the manner in which they are obtained as described above, will become more apparent and be more clearly understood in connection with the following description of exemplary embodiments, which are explained with reference to the accompanying drawings.

### BRIEF DESCRIPTION OF THE DRAWINGS

In the drawings, same element numbers indicate same elements in each of the views:
- Figure 1: is a schematic cross-sectional illustration of an exemplary e-cigarette;
- Figures 2 to 7: show an exemplary embodiment of an e-cigarette in ready-for-use and in refill states;
- Figures 8 to 10: depict the exemplary embodiment of figures 2 to 7 in a schematic cross-sectional view;
- Figure 11: illustrates the exemplary embodiment of figures 2 to 10 in another schematic cross-sectional view; and
- Figure 12: illustrates the exemplary embodiment of figures 2 to 11 in another schematic cross-sectional view.

### DESCRIPTION OF THE PREFERRED EMBODIMENTS

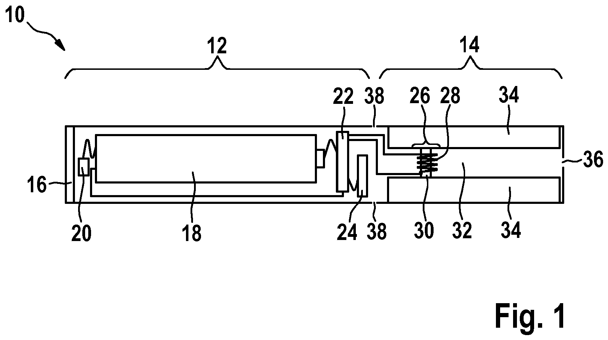

Throughout the following, an electronic smoking device will be exemplarily described with reference to an e-cigarette. As is shown in Figure 1, an e-cigarette 10 typically has a housing comprising a cylindrical hollow tube having an end cap 16. The cylindrical hollow tube may be a single-piece or a multiple-piece tube. In Figure 1, the cylindrical hollow tube is shown as a two-piece structure having a power supply portion 12 and an atomizer/liquid reservoir portion 14. Together the power supply portion 12 and the atomizer/liquid reservoir portion 14 form a cylindrical tube which can be approximately the same size and shape as a conventional cigarette, typically about 100 mm with a 7.5 mm diameter, although lengths may range from 70 to 150 or 180 mm, and diameters from 5 to 28 mm.

The power supply portion 12 and atomizer/liquid reservoir portion 14 are typically made of metal, e.g. steel or aluminum, or of hardwearing plastic and act together with the end cap 16 to provide a housing to contain the components of the e-cigarette 10. The power supply portion 12 and an atomizer/liquid reservoir portion 14 may be configured to fit together by a friction push-fit, a snap-fit, or a bayonet attachment, magnetic-fit, or screw threads. The end cap 16 is provided at the front end of the power supply portion 12. The end cap 16 may be made from translucent plastic or other translucent material to allow a light-emitting diode (LED) 20 positioned near the end cap to emit light through the end cap. The end cap can be made of metal or other materials that do not allow light to pass.

An air inlet may be provided in the end cap, at the edge of the inlet next to the cylindrical hollow tube, anywhere along the length of the cylindrical hollow tube, or at the connection of the power supply portion 12 and the atomizer/liquid reservoir portion 14. Figure 1 shows a pair of air inlets 38 provided at the intersection between the power supply portion 12 and the atomizer/liquid reservoir portion 14.

A power supply, preferably a battery 18, an LED 20, control electronics 22 and optionally an airflow sensor 24 are provided within the cylindrical hollow tube power supply portion 12. The battery 18 is electrically connected to the control electronics 22, which are electrically connected to the LED 20 and the airflow sensor 24. In this example, the LED 20 is at the front end of the power supply portion 12, adjacent to the end cap 16 and the control electronics 22 and airflow sensor 24 are provided in the central cavity at the other end of the battery 18 adjacent to the atomizer/liquid reservoir portion 14.

The airflow sensor 24 acts as a puff detector, detecting a user puffing or sucking on the atomizer/liquid reservoir portion 14 of the e-cigarette 10. The airflow sensor 24 can be any suitable sensor for detecting changes in airflow or air pressure, such as a microphone switch including a deformable membrane which is caused to move by variations in air pressure. Alternatively, the sensor may be a Hall element or an electro-mechanical sensor.

The control electronics 22 are also connected to an atomizer 26. In the example shown, the atomizer 26 includes a heating coil 28 which is wrapped around a wick 30 extending across a central passage 32 of the atomizer/liquid reservoir portion 14. The coil 28 may be positioned anywhere in the atomizer 26 and may be transverse or parallel to the liquid reservoir 34. The wick 30 and heating coil 28 do not completely block the central passage 32. Rather an air gap is provided on either side of the heating coil 28 enabling air to flow past the heating coil 28 and the wick 30. The atomizer may alternatively use other forms of heating elements, such as ceramic heaters, or fiber or mesh material heaters. Nonresistance heating elements such as sonic, piezo and jet spray may also be used in the atomizer in place of the heating coil.

The central passage 32 is surrounded by a cylindrical liquid reservoir 34 with the ends of the wick 30 abutting or extending into the liquid reservoir 34. The wick 30 may be a porous material such as a bundle of fiberglass fibers, with liquid in the liquid reservoir 34 drawn by capillary action from the ends of the wick 30 towards the central portion of the wick 30 encircled by the heating coil 28.

The liquid reservoir 34 may alternatively include wadding soaked in liquid which encircles the central passage 32 with the ends of the wick 30 abutting the wadding. In other examples, the liquid reservoir 34 may comprise a toroidal cavity arranged to be filled with liquid and with the ends of the wick 30 extending into the toroidal cavity.

An air inhalation port 36 is provided at the back end of the atomizer/liquid reservoir portion 14 remote from the end cap 16. The inhalation port 36 may be formed from the cylindrical hollow tube atomizer/liquid reservoir portion 14 or maybe formed in an end cap.

In use, a user sucks on the e-cigarette 10. This causes air to be drawn into the e-cigarette 10 via one or more air inlets, such as air inlets 38, and to be drawn through the central passage 32 towards the air inhalation port 36. The change in air pressure which arises is detected by the airflow sensor 24, which generates an electrical signal that is passed to the control electronics 22. In response to the signal, the control electronics 22 activate the heating coil 28, which causes liquid present in the wick 30 to be vaporized, creating an aerosol (which may comprise gaseous and liquid components) within the central passage 32. As the user continues to suck on the e-cigarette 10, this aerosol is drawn through the central passage 32 and inhaled by the user. At the same time, the control electronics 22 also activate the LED 20 causing the LED 20 to light up which is visible via the translucent end cap 16 mimicking the appearance of a glowing ember at the end of a conventional cigarette. As liquid present in the wick 30 is converted into an aerosol, more liquid is drawn into the wick 30 from the liquid reservoir 34 by capillary action and thus is available to be converted into an aerosol through subsequent activation of the heating coil 28.

Some e-cigarettes are intended to be disposable and the electric power in the battery 18 is intended to be sufficient to vaporize the liquid contained within the liquid reservoir 34, after which the e-cigarette 10 is thrown away. In other examples the battery 18 is rechargeable and the liquid reservoir 34 is refillable. In the cases where the liquid reservoir 34 is a toroidal cavity, this may be achieved by refilling the liquid reservoir 34 via a refill port. In other examples the atomizer/liquid reservoir portion 14 of the e-cigarette 10 is detachable from the power supply portion 12 and a new atomizer/liquid reservoir portion 14 can be fitted with a new liquid reservoir 34 thereby replenishing the supply of liquid. In some cases, replacing the liquid reservoir 34 may involve replacement of the heating coil 28 and the wick 30 along with the replacement of the liquid reservoir 34. A replaceable unit comprising the atomizer 26 and the liquid reservoir 34 is called a cartomizer.

The new liquid reservoir 34 may be in the form of a cartridge having a central passage 32 through which a user inhales aerosol. In other examples, aerosol may flow around the exterior of the cartridge 32 to an air inhalation port 36.

Of course, in addition to the above description of the structure and function of a typical e-cigarette 10, variations also exist. For example, the LED 20 may be omitted. The airflow sensor 24 may be placed adjacent to the end cap 16 rather than in the middle of the e-cigarette. The airflow sensor 24 may be replaced with a switch which enables a user to activate the e-cigarette manually rather than in response to the detection of a change in air flow or air pressure. Different types of atomizers may be used. Thus, for example, the atomizer may have a heating coil in a cavity in the interior of a porous body soaked in liquid. In this design aerosol is generated by evaporating the liquid within the porous body either by activation of the coil heating the porous body or alternatively by the heated air passing over or through the porous body. Alternatively, the atomizer may use a piezoelectric atomizer to create an aerosol either in combination or in the absence of a heater.

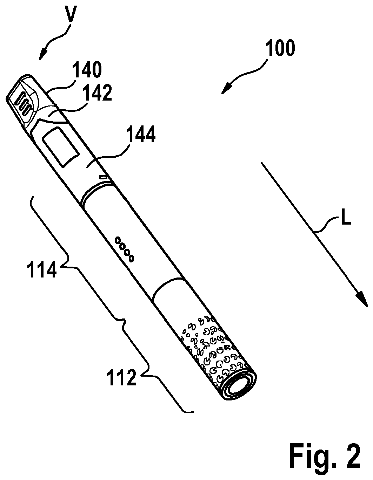

Figures 2 to 7 show an embodiment of the e-cigarette 100 in ready-to-use and refill states. The e-cigarette 100 comprises the power supply portion 112 and the atomizer/liquid reservoir portion 114.

Optionally, the power supply portion 112 is removable from the atomizer/liquid reservoir portion 114 or vice versa.

Figure 2 shows the c-cigarette 100 in a ready-to-use state, in which a user of the e-cigarette 100 can use the e-cigarette 100 for vaping, i.e. for inhaling the atomized liquid storable in the atomizer/liquid reservoir portion 114. In the ready-to-use state, a mouthpiece cap 140 of the e-cigarette 100 is arranged in its vaping position V, in which the mouthpiece cap 140 borders, i.e. directly contacts, the atomizer/liquid reservoir portion 114, such that an outer side 142 of the mouthpiece cap 140 forms an essentially continuous surface with an outer side 144 of the atomizer/liquid reservoir portion 114.

The atomizer/liquid reservoir portion 114 of the exemplary embodiment of figure 2 is arranged between the power supply portion 112 and the mouthpiece cap 140 in a longitudinal direction L of the e-cigarette 100, the longitudinal direction L pointing from the mouthpiece cap 140 to the power supply portion 112.

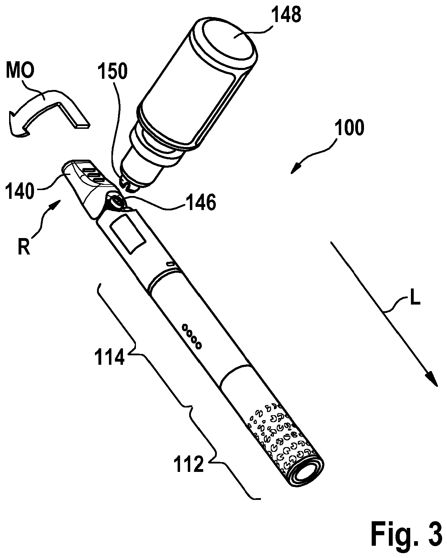

Figure 3 shows the exemplary embodiment of figure 2 in a refill state. In the refill state, the mouthpiece cap 140 is arranged in its refill position R. In the refill position R, a docking valve 146 of the e-cigarette 100 for receiving liquid to be stored in the atomizer/liquid reservoir portion 114 is exposed or uncovered by the mouthpiece cap 140.

In its refill position R, the mouthpiece cap 140 is arranged at a distance to its vaping position V at least against the longitudinal direction L of the e-cigarette 100. Hence, in the refill position R, the mouthpiece cap 140 is arranged further away from the power supply portion 112 compared to its vaping position V.

Optionally or additionally, the mouthpiece cap 140 may be rotated around the longitudinal direction L by a predefined angle compared to its vaping position V. In the exemplary embodiment of figure 3, the mouthpiece cap 140 is arranged further away from the power supply portion 112 and rotated around the longitudinal direction L with respect to the vaping position V shown in figure 2, in which the mouthpiece cap 140 covers the docking valve 146, such that dirt or dust is hindered from entering the docking valve 146.

The arrow MO indicates a possible movement path of the mouthpiece cap 140 between the refill position R and the vaping position V.

At least in the refill position R and optionally also in the vaping position V, the mouthpiece cap 140 is captively or undetachably connected to the atomizer/liquid reservoir portion 114, such that the mouthpiece cap 140 cannot be lost.

In the exposed or uncovered state of the docking valve 146, a source of liquid, exemplarily shown as a refill bottle 148, can dock at the docking valve 146 in order to refill the e-cigarette 100, in particular the liquid reservoir of the e-cigarette 100. In order to dock the refill bottle 148 with the docking valve 146, a counter docking valve 150 of the refill bottle 148 can be placed onto or inside of the docking valve 146, e.g. essentially perpendicular to the longitudinal direction L of the e-cigarette 100.

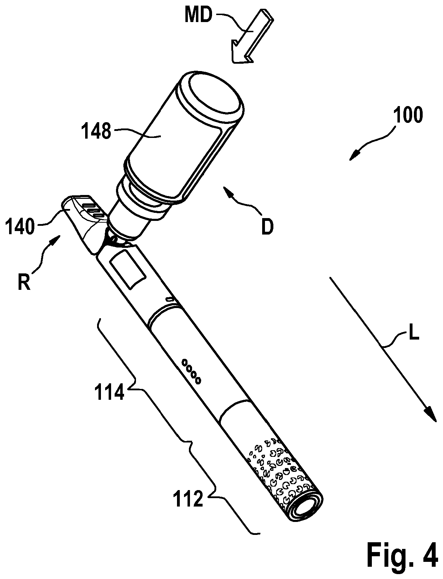

Figure 4 shows the exemplary embodiment of figures 2 and 3, wherein the refill bottle 148 is in a docked position D after the refill bottle 148 has been moved along a movement path for docking MD, which essentially extends perpendicularly to the longitudinal direction L.

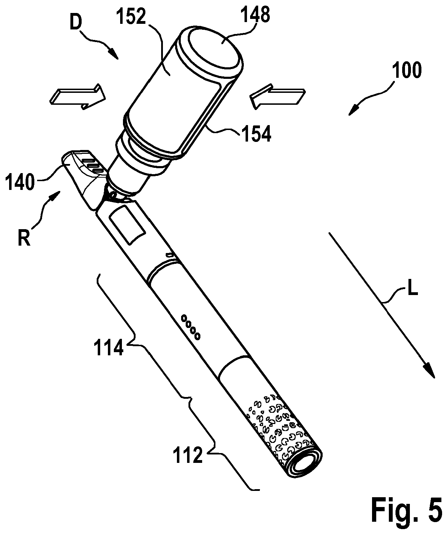

Figure 5 shows the refill bottle 148 in its docked position D. In order to refill the e-cigarette 100, the refill bottle 148 can be squeezed, e.g. perpendicular the longitudinal direction L. Squeezing the refill bottle 148 means compressing the refill bottle 148, or pressing opposite side walls 152, 154 of the refill bottle 148 together. In the docked position D, the side walls 152, 154 can extend essentially perpendicularly to the longitudinal direction L of the e-cigarette 100.

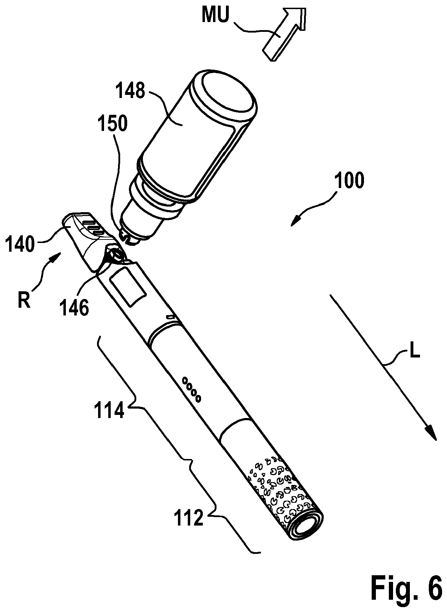

Figure 6 shows the exemplary embodiment of figures 2 to 5. In order to separate the refill bottle 148 from the e-cigarette 100, the refill bottle 148 is moved away from the e-cigarette 100 along the movement path for undocking MU. In order to release the connection in between the docking valve 146 and the counter docking valve 150, at least the movement along the movement path for undocking MU is sufficient. Optionally, the refill bottle 148 may additionally tilted back and forth perpendicularly to the movement path for undocking MU.

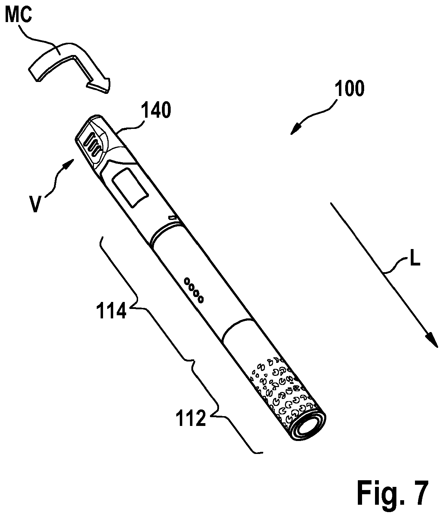

Figure 7 shows the exemplary embodiment of figures 2 to 6. The mouthpiece cap 140 is shown positioned in its vaping position V. The vaping position V may differ from the refill position R along and/or around the longitudinal direction L. In the exemplary embodiment of figure 7, the mouthpiece cap 140 is moved from the refill position R to the vaping position V along the movement path MC, i.e. first around the longitudinal direction L and, then, along the longitudinal direction L.

The exemplary embodiment of the electronic smoking device comprising the mouthpiece cap 140 and the docking valve 146 may, as such, be advantages on its own and independent of the further features of the exemplary embodiment mentioned in the following.

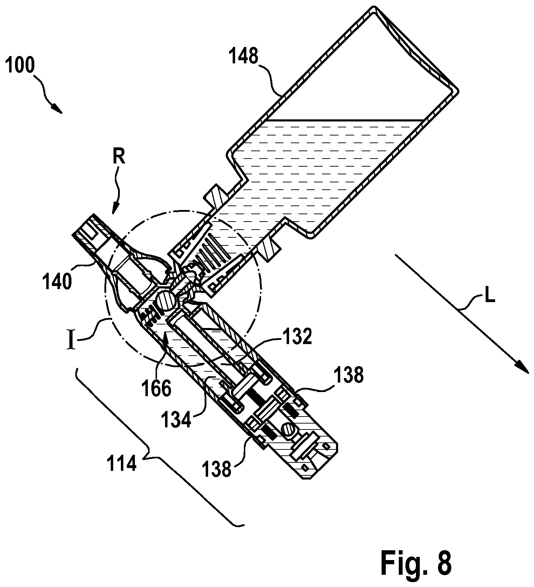

Figure 8 shows the exemplary embodiment of figures 2 to 7 in a cross-sectional view, wherein the cross-section extends along the longitudinal direction L of the e-cigarette 100.

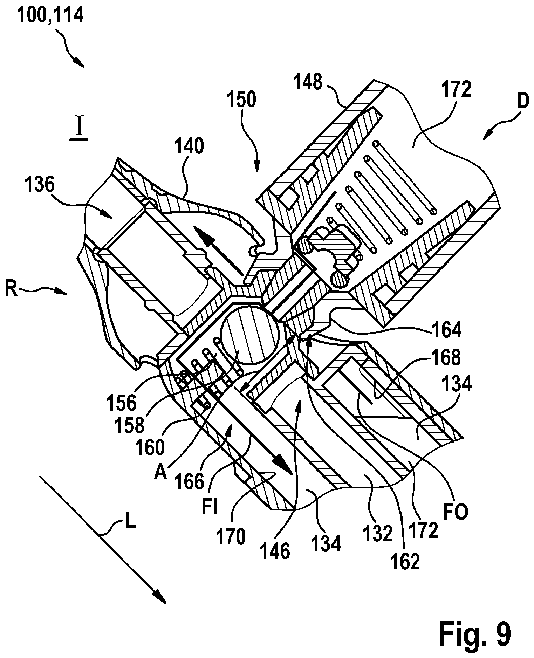

Figure 9 shows an enlarged detail I of figure 8.

Figures 8 and 9 show the liquid reservoir 134 of the atomizer/liquid reservoir portion 114. A central passage 132 of the atomizer/liquid reservoir portion 114 extends through the liquid reservoir 134 against the longitudinal direction L in order to transport atomized liquid towards an air inhalation port 136 of the atomizer/liquid reservoir portion 114, the air inhalation port 136 opening into the mouthpiece cap 140. The liquid reservoir 134 has a cylindrical cross-section and extends along the longitudinal direction L. The central passage 132 is arranged in the liquid reservoir 134, such that the liquid reservoir 134 surrounds or encompasses the central passage 132 at least sectionwise or even completely perpendicular to the longitudinal direction L.

The atomizer/liquid reservoir portion 114 is formed with a liquid duct 156, that is adapted to the receive liquid from the docking valve 146 and that opens into the liquid reservoir 134 at a distance A to the docking valve 146. The liquid duct 156 essentially extends perpendicularly to the longitudinal direction L. Hence, sections of the liquid reservoir 134, which are arranged at a distance from the docking valve 146 can receive liquid first when refilling the liquid reservoir 134. Thus, air present in the liquid reservoir 134 before refilling the liquid reservoir 134 can escape at least along a part of the liquid reservoir 134 that is arranged at the liquid duct 156 and/or the docking valve 146.

The docking valve 146 is shown with a sealing element 158 that is pressed into a sealing position, in which the sealing element 158 seals the docking valve 146. The docking valve 146 comprises a resilient element 160 that seeks to press the sealing element 158 in the sealed position.

For example, the sealing element 158 has a spherical shape, e.g. the shape of a ball. The resilient element 160 is e.g. a spring and for example a coil spring.

The resilient element 160 in particular seeks to press the sealing element 156 perpendicular to the longitudinal direction L into its sealing position.

In the embodiment shown in figures 8 and 9, the mouthpiece cap 140 is positioned in its refill position R and the counter docking valve 150 of the refill bottle 148 is docked to the docking valve 146. The counter docking valve 150 presses the sealing element 158 out of its sealing position against the spring force of the resilient element 160, i.e. essentially perpendicular to the longitudinal direction L.

The docking valve 146 is adapted to form a snap-fit connection with the counter docking valve 150 of the refill bottle 148. For example, the docking valve 146 has a docking section, wherein the docking section comprises a convex- or a concave-shaped cross-section, thereby forming a setback or recess 162. The counter docking valve 150 is adapted to form a snap-fit connection with the docking valve 146 of the electronic smoking device. The counter docking valve 150 for example, comprises elastic latch claws 164 that are arranged around a central axis of the counter docking valve 150. The latch claws 164 are shown curved towards the central axis and are adapted to engage the recess 162 in the docked position D of the refill bottle 148.

The embodiment of the docking valve 148 forming the recess 162 and the counter docking valve 150 with the elastic latch claws 164 is advantages on its own and in particular independent of the embodiments discussed previously and in the following.

The liquid duct 156 opens into the liquid reservoir 134 with an outlet orifice 166. The outlet orifice 166 is arranged at a predetermined distance to a side wall section 168 of the liquid reservoir 134 at which the docking valve 146 is arranged. For example, the outlet orifice 166 is arranged closer to a side wall section 170 of the liquid reservoir 134 opposite to the side wall section 168, i.e. opposite to the docking valve 146, than to the docking valve 146. In particular, the outlet orifice 166 opens into a section of the liquid reservoir 134 that is opposite to another section of the liquid reservoir 134 arranged at the docking valve 146.

Arrow Fl indicates the flow-in path of liquid 172 flowing from the refill bottle 148 into the liquid reservoir 134. The other arrow FO indicates a flow-out path of air as it escapes out of the liquid reservoir 134 when refilling the liquid 172. The flow-in path FI begins inside of the refill bottle 148 and extends through the counter docking valve 150, the docking valve 146 and through the liquid duct 156 into the liquid reservoir 134. The flow-out path FO extends through a part of the liquid reservoir 134 that surrounds the liquid duct 156 and/or is arrange at the docking valve 146 against the longitudinal direction L towards the air inhalation port 136. Along the air inhalation port 136, the flow-out path FO extends through the mouthpiece cap 140 outside the electronic smoking device 100. The embodiment of the atomizer/liquid reservoir portion 114 with the liquid duct 156 and optionally with the outlet orifice 166 is advantages on its own independent of the embodiments previously and in the following.

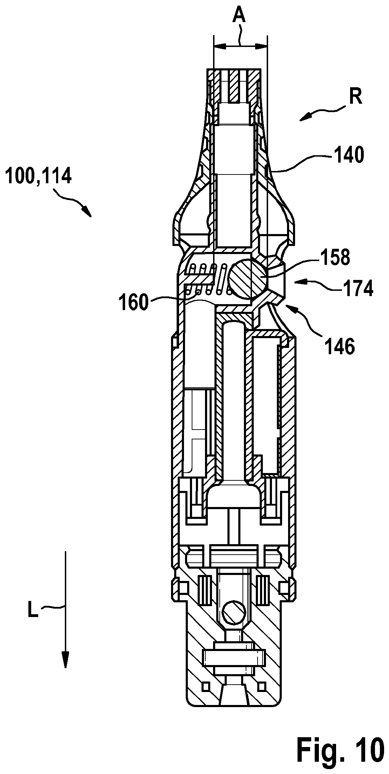

Figure 10 shows the exemplary embodiment of figures 2 to 9 in the cross-sectional view of figures 8 and 9, wherein no refill bottle is docked with the docking valve 146. The sealing element 158 seals the docking valve 146, wherein the resilient element 160 presses the sealing element 158 against side walls of the docking valve 146 that delimit an inlet opening 174 of the docking valve 146.

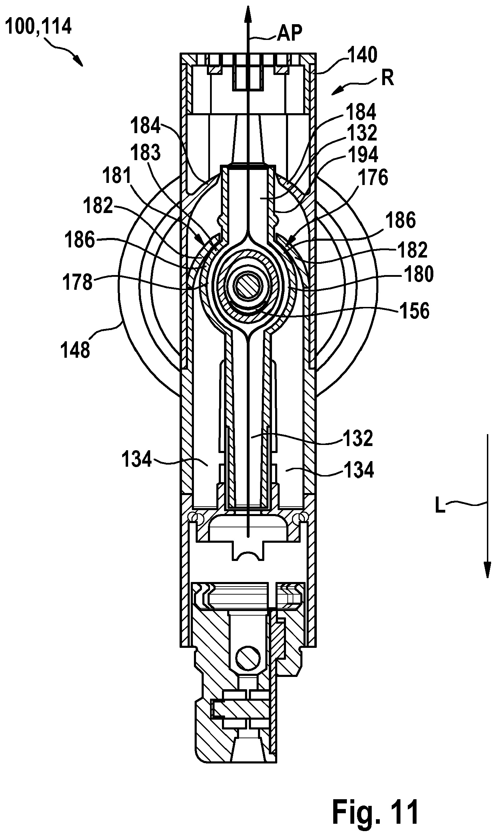

Figure 11 shows the exemplary embodiment of figures 2 to 10 in another cross-sectional view, wherein the cross-section extends along the longitudinal direction L. The docking valve 146 points into the drawing plane of figure 11.

The cross-sectional plane extends along the longitudinal direction L through the central passage 132. The liquid duct 156 extends through the central passage 132, in particular, through a convex section 176 of the central passage 132. The convex section 176 is formed by two passage sections 178, 180 that interconnect parts of the central passage 132 before and after the liquid duct 156 in the longitudinal direction L. The passage sections 178, 180 essentially form a passage ring through which the liquid duct 156 extends essentially perpendicular to the longitudinal direction L. The path of atomized liquid extends through the central passage 132 and through the passage sections 178, 180 is indicated by the arrow AP.

According to the invention, the atomizer/liquid reservoir portion 114 comprises a valve 181 for releasing gas, e.g. air from the liquid reservoir 134 when refilling the liquid reservoir 134, the valve 181 comprising a flexible sealing element 182 and essentially rigid counter sealing element 186 formed by an outer side wall section 183 of the central passage 132.

The section of the central passage 132 that provides for the rigid counter sealing element 186 has a curved shape.

The atomizer/liquid reservoir portion 114 comprises at least one flexible sealing element 182 that rests against the outer side wall section 183 of the convex section 176 and thereby closes the flow-out path FO. When refilling the atomizer/liquid reservoir portion 114, pressing the liquid out of the refill bottle 148 into the liquid reservoir 134 increases the pressure of air within the liquid reservoir 134. This increased pressure is sufficient in order to replace or deform the flexible sealing element 182, such that air can escape from the liquid reservoir 134 via the flexible sealing element 182 towards the mouthpiece cap 140.

The flexible sealing element 182 can be an umbrella seal or can comprise at least one flexible sealing tongue or lip with a fixed end in the longitudinal direction L and a free end against the longitudinal direction L.

The mouthpiece cap 140 can comprise at least one closing element 184 that forces the flexible sealing element 182 against a rigid counter sealing element 186 provided by the convex section 176 and in particular by the outer side wall section 183. The rigid counter sealing element 186 and the flexible sealing element 182 are shown arranged at a side of the convex section 176 that faces the mouthpiece cap 140.

In the refill position R the closing element 184 is arranged at a distance to the flexible sealing element 182 against the longitudinal direction L. In the vaping position V of the mouthpiece cap 140, the closing element 184 rests against the flexible sealing element 182 and holds the flexible element 182 against the rigid counter sealing element 186.

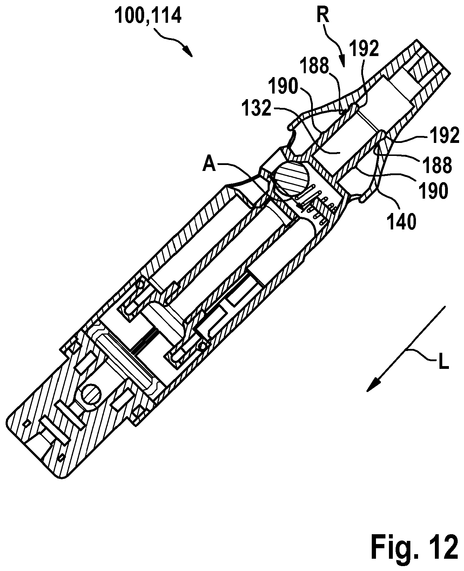

The embodiment of the atomizer/liquid reservoir portion 114 with the flexible sealing element 182, the closing element 184 and the rigid counter sealing element 186 as such is advantages on its own and independent of the embodiments disclosed previously and in the following. Figure 12 shows the exemplary embodiment of figures 2 to 11 in the cross-sectional view of figure 10.

The mouthpiece cap 140 is held in the refill position R and/or the vaping position V in a force-fit manner. For example, the mouthpiece cap 140 is held in the refill position R or in the vaping position V by a latched connection. A latch element 188, e.g. a protrusion, is provided by an end of the closing element 184 perpendicular to the longitudinal direction L, and counter latch elements 190, 192, for example latch protrusions or latch recesses, are provided by an outer side wall section 194 of the central passage 132. The outer side wall section 194 may be the lateral surface of the central passage 132. The counter latch element 190 that holds the mouthpiece cap 140 in the vaping position V may be provided by a latch protrusion and the counter latch element 192 that holds the mouthpiece cap 140 in the refill position R may be provided by a latch recess or a set back provides by the latch recess or a protrusion.

### LIST OF REFERENCE SIGNS

- 10, 100: electronic smoking device
- 12, 112: power supply portion
- 14, 114: atomizer/liquid reservoir portion
- 16: end cap
- 18: battery
- 20: light-emitting diode (LED)
- 22: control electronics
- 24: airflow sensor
- 26, 126: atomizer
- 28: heating coil
- 30: wick
- 32, 132: central passage
- 34, 134: liquid reservoir
- 36, 136: air inhalation port
- 38, 138: air inlets
- 140: mouthpiece cap
- 142: outer side of mouthpiece cap
- 144: outer side of atomizer/liquid reservoir portion
- 146: docking valve
- 148: refill bottle
- 150: counter docking valve
- 152, 154: side walls of refill bottle
- 156: liquid duct
- 158: sealing element
- 160: resilient element
- 162: recess
- 164: elastic latch claws
- 166: outlet orifice of liquid duct
- 168: side wall section of liquid reservoir with docking valve
- 170: side wall section of liquid reservoir opposite to docking valve
- 172: liquid
- 174: inlet opening of docking valve
- 176: convex section of central passage
- 178, 180: passage section
- 181: valve
- 182: flexible sealing element
- 183: outer side wall section of convex section of central passage
- 184: closing element
- 186: rigid counter sealing element
- 188: latch element
- 190, 192: counter latch element
- 194: outer side wall section of central passage with counter latch element

- A: distance
- AP: path of atomized liquid
- D: docked position
- FI: flow-in path
- FO: flow-out path
- L: longitudinal direction
- MC: movement path from vaping to refill position
- MD: movement path for docking
- MO: movement path from refill to vaping position
- MU: movement path for undocking
- R: refill position
- V: vaping position

## Claims

1. An electronic smoking device (100), comprising:
a power supply portion (112) comprising a power supply (18), and
an atomizer/liquid reservoir portion (114) comprising
a refillable liquid reservoir (134) adapted for storing liquid (172),
an atomizer (26) operable when connected to the power supply (18) to atomize liquid (172) stored in the liquid reservoir (134), and
a central passage (132) that is adapted to conduct atomized liquid, the central passage (132) extending through the refillable liquid reservoir (134),
wherein the atomizer/liquid reservoir portion (114) comprises
a docking valve (146) for docking a source of liquid (148) to be stored in the liquid reservoir (134),
a liquid duct (156) that is adapted to receive liquid (172) from the docking valve (146) and that opens into the refillable liquid reservoir (134) at a predetermined distance (A) to the docking valve (146), and
a valve (181) for releasing gas from the refillable liquid reservoir (134) when refilling the refillable liquid reservoir (134), the valve (181) comprising a flexible sealing element (182) and an essentially rigid counter sealing element (186) formed by an outer side wall section (183) of the central passage (132).

2. The electronic smoking device (100) according to claim 1, wherein the liquid duct (156) opens into the refillable liquid reservoir (134) with an outlet orifice (166).

3. The electronic smoking device (100) according to claim 2, wherein the outlet orifice (166) is arranged closer to a side wall section (170) of the refillable liquid reservoir (134) opposite to the docking valve (146) than to the docking valve (146).

4. The electronic smoking device (100) according to claim 1, wherein the section of the central passage (132) that provides for the rigid counter sealing element (186) has a curved shape.

5. The electronic smoking device (100) according to claim 1 or 4, wherein the liquid duct (156) extends essentially perpendicular to and through the central passage (132) at a convex section (176) of the central passage (132).

6. The electronic smoking device (100) according to any one of claims 1 to 5, wherein the electronic smoking device (100) comprises a mouthpiece cap (140) in communication with an air inhalation port (136) for providing atomized liquid to a user, wherein the mouthpiece cap (140) comprises a refill position (R) and a vaping position (V), wherein in the vaping position (V), the mouthpiece cap (140) is closer to an opposite end of the electronic smoking device (100) than in the refill position (R), and/or wherein in the vaping position (V,) the mouthpiece cap (140) is rotated around a longitudinal axis of the electronic smoking device (100) by a predetermined angle compared to the refill position (R).

7. The electronic smoking device (100) according to claim 6, wherein the docking valve (146) is covered by the mouthpiece cap (140) in the vaping position (V), and is accessible when the mouthpiece cap (140) is in the refill position (R).

8. The electronic smoking device (100) according to claim 6 or 7, wherein the mouthpiece cap (140) comprises a closing element (184) that forces the flexible sealing element (182) against the rigid counter sealing element (186) when the mouthpiece cap (140) is in the vaping position (V).

9. The electronic smoking device (100) according to claim 8, wherein the closing element (184) is arranged at a distance to the flexible sealing element (182) when the mouthpiece cap (140) is in the refill position (R).

10. The electronic smoking device (100) according to any one of claims 6 to 9, wherein the mouthpiece cap (140) is held in the refill position (R) and/or in the vaping position (V) in a force-fit manner and/or in a form-fit manner.

11. The electronic smoking device (100) according to any one of claims 8 to 9, wherein a latch element (188) of a latch connection holding the mouthpiece cap (140) in the vaping position (V) and/or in the refill position (R) is provided by the closing element (184), and a counter latch element (190, 192) is provided by an outer side wall section (194) of the central passage (132).

12. The electronic smoking device (100) according to any one of claims 1 to 11, wherein the docking valve (146) is adapted to form a snap-fit connection with a counter docking valve (150) of a source of liquid.

13. The electronic smoking device (100) according to any of claims 1 to 12, wherein the docking valve (146) has a docking section, the docking section comprising a convex or a concave shape.

14. An atomizer/liquid reservoir portion (114) comprising:
a liquid reservoir (134),
an atomizer (26) operable when connected to a power supply (18) for an electronic smoking device (100) to atomize liquid stored in the liquid reservoir (134), and
a central passage (132) that is adapted to conduct atomized liquid, the central passage (132) extending through the refillable liquid reservoir (134),
wherein the atomizer/liquid reservoir portion (114) comprises
a docking valve (146) for docking a source of liquid (148) to be stored in the liquid reservoir (134),
a liquid duct (156) that is adapted to receive liquid (172) from the docking valve (146) and that opens into the refillable liquid reservoir (134) at a predetermined distance (A) to the docking valve (146), and
a valve (181) for releasing gas from the refillable liquid reservoir (134) when refilling the refillable liquid reservoir (134), the valve (181) comprising a flexible sealing element (182) and an essentially rigid counter sealing element (186) formed by an outer side wall section (183) of the central passage (132).

## Patentansprüche

1. Elektronische Rauchvorrichtung (100), umfassend:
einen Stromversorgungsabschnitt (112), der eine Stromversorgung (18) umfasst, und
einen Zerstäuber-/Flüssigkeitsreservoirabschnitt (114), umfassend
ein nachfüllbares Flüssigkeitsreservoir (134), das dazu ausgelegt ist, eine Flüssigkeit (172) zu speichern,
einen Zerstäuber (26), der, wenn er mit der Stromversorgung (18) verbunden ist, dazu betreibbar ist, in dem Flüssigkeitsbehälter (134) gespeicherte Flüssigkeit (172) zu zerstäuben, und
einen zentralen Durchgang (132), der dazu ausgelegt ist, zerstäubte Flüssigkeit zu führen, wobei sich der zentrale Durchgang (132) durch das nachfüllbare Flüssigkeitsreservoir (134) erstreckt,
wobei der Zerstäuber-/Flüssigkeitsreservoirabschnitt (114) Folgendes umfasst:
ein Andockventil (146) zum Andocken einer Quelle von in dem Flüssigkeitsreservoir (134) zu speichernder Flüssigkeit (148),
einen Flüssigkeitskanal (156), der dazu ausgelegt ist, Flüssigkeit (172) von dem Andockventil (146) zu empfangen, und mit einem vorbestimmten Abstand (A) zu dem Andockventil (146) in das nachfüllbare Flüssigkeitsreservoir (134) mündet, und
ein Ventil (181) zum Abgeben von Gas aus dem nachfüllbaren Flüssigkeitsreservoir (134) beim Nachfüllen des nachfüllbaren Flüssigkeitsreservoirs (134), wobei das Ventil (181) ein flexibles Dichtungselement (182) und ein von einem äußeren Seitenwandbereich (183) des zentralen Durchgangs (132) ausgebildetes, im Wesentlichen starres Gegendichtungselement (186) umfasst.

2. Elektronische Rauchvorrichtung (100) nach Anspruch 1, wobei der Flüssigkeitskanal (156) mit einer Austrittsöffnung (166) in das nachfüllbare Flüssigkeitsreservoir (134) mündet.

3. Elektronische Rauchvorrichtung (100) nach Anspruch 2, wobei die Austrittsöffnung (166) näher an einem Seitenwandbereich (170) des nachfüllbaren Flüssigkeitsreservoirs (134) gegenüber dem Andockventil (146) angeordnet ist als an dem Andockventil (146).

4. Elektronische Rauchvorrichtung (100) nach Anspruch 1, wobei der Bereich des zentralen Durchgangs (132), der das starre Gegendichtungselement (186) bereitstellt, eine gebogene Form aufweist.

5. Elektronische Rauchvorrichtung (100) nach Anspruch 1 oder 4, wobei sich der Flüssigkeitskanal (156) in einem konvexen Bereich (176) des zentralen Durchgangs (132) im Wesentlichen senkrecht zu dem zentralen Durchgang (132) und durch diesen hindurch erstreckt.

6. Elektronische Rauchvorrichtung (100) nach einem der Ansprüche 1 bis 5, wobei die elektronische Rauchvorrichtung (100) eine Mundstückkappe (140) in Kommunikation mit einer Luftinhalationsöffnung (136) zum Bereitstellen von zerstäubter Flüssigkeit an einen Benutzer umfasst, wobei die Mundstückkappe (140) eine Nachfüllposition (R) und eine Vapingposition (V) umfasst, wobei die Mundstückkappe (140) sich in der Vapingposition (V) näher an einem entgegengesetzten Ende der elektronischen Rauchvorrichtung (100) befindet als in der Nachfüllposition (R),
und/oder wobei die Mundstückkappe (140) in der Vapingposition (V) um eine Längsachse der elektronischen Rauchvorrichtung (100) um einen vorbestimmten Winkel, verglichen mit der Nachfüllposition (R), gedreht ist.

7. Elektronische Rauchvorrichtung (100) nach Anspruch 6, wobei das Andockventil (146) in der Vapingposition (V) von der Mundstückkappe (140) verdeckt wird und zugänglich ist, wenn sich die Mundstückkappe (140) in der Nachfüllposition (R) befindet.

8. Elektronische Rauchvorrichtung (100) nach Anspruch 6 oder 7, wobei die Mundstückkappe (140) ein Schließelement (184) umfasst, welches das flexible Dichtungselement (182) gegen das starre Gegendichtungselement (186) drückt, wenn die Mundstückkappe (140) sich in der Vapingposition (V) befindet.

9. Elektronische Rauchvorrichtung (100) nach Anspruch 8, wobei das Schließelement (184) mit einem Abstand zu dem flexiblen Dichtungselement (182) angeordnet ist, wenn sich die Mundstückkappe (140) in der Nachfüllposition (R) befindet.

10. Elektronische Rauchvorrichtung (100) nach einem der Ansprüche 6 bis 9, wobei die Mundstückkappe (140) auf kraftschlüssige Weise und/oder auf formschlüssige Weise in der Nachfüllposition (R) und/oder in der Vapingposition (V) gehalten wird.

11. Elektronische Rauchvorrichtung (100) nach einem der Ansprüche 8 bis 9, wobei ein Rastelement (188) einer Rastverbindung, welche die Mundstückkappe (140) in der Vapingposition (V) und/oder in der Nachfüllposition (R) hält, von dem Schließelement (184) bereitgestellt wird und ein Gegenrastelement (190, 192) von einem äußeren Seitenwandbereich (194) des zentralen Durchgangs (132) bereitgestellt wird.

12. Elektronische Rauchvorrichtung (100) nach einem der Ansprüche 1 bis 11, wobei das Andockventil (146) dazu ausgelegt ist, mit einem Gegenandockventil (150) einer Flüssigkeitsquelle eine Schnappverschlussverbindung auszubilden.

13. Elektronische Rauchvorrichtung (100) nach einem der Ansprüche 1 bis 12, wobei das Andockventil (146) einen Andockbereich aufweist, wobei der Andockbereich eine konvexe oder konkave Form umfasst.

14. Zerstäuber-/Flüssigkeitsreservoirabschnitt (114), umfassend:
ein Flüssigkeitsreservoir (134),
einen Zerstäuber (26), der, wenn er mit einer Stromversorgung (18) für eine elektronische Rauchvorrichtung (100) verbunden ist, zum Zerstäuben von in dem Flüssigkeitsreservoir (134) gespeicherter Flüssigkeit betreibbar ist, und
einen zentralen Durchgang (132), der dazu ausgelegt ist, zerstäubte Flüssigkeit zu leiten, wobei sich der zentrale Durchgang (132) durch das nachfüllbare Flüssigkeitsreservoir (134) erstreckt,
wobei der Zerstäuber-/Flüssigkeitsreservoirabschnitt (114) Folgendes umfasst:
ein Andockventil (146) zum Andocken einer Quelle von in dem Flüssigkeitsreservoir (134) zu speichernder Flüssigkeit (148),
einen Flüssigkeitskanal (156), der dazu ausgelegt ist. Flüssigkeit (172) von dem Andockventil (146) zu empfangen, und mit einem vorbestimmten Abstand (A) zu dem Andockventil (146) in das nachfüllbare Flüssigkeitsreservoir (134) mündet, und
ein Ventil (181) zum Abgeben von Gas aus dem nachfüllbaren Flüssigkeitsreservoir (134) beim Nachfüllen des nachfüllbaren Flüssigkeitsreservoirs (134), wobei das Ventil (181) ein flexibles Dichtungselement (182) und ein von einem äußeren Seitenwandbereich (183) des zentralen Durchgangs (132) ausgebildetes, im Wesentlichen starres Gegendichtungselement (186) umfasst.

## Revendications

1. Dispositif de cigarette électronique(100), comprenant :
une portion d'alimentation électrique (112) comprenant une alimentation électrique (18), et
une portion d'atomiseur/réservoir de liquide (114) comprenant
un réservoir de liquide rechargeable (134) adapté pour stocker du liquide (172), un atomiseur (26) utilisable lorsqu'il est connecté à l'alimentation électrique (18) pour atomiser du liquide (172) stocké dans le réservoir de liquide (134), et
un passage central (132) qui est adapté pour conduire du liquide atomisé, le passage central (132) s'étendant à travers le réservoir de liquide rechargeable (134),
la portion d'atomiseur/réservoir de liquide (114) comprenant
une soupape d'accueil (146) pour accueillir une source de liquide (148) à stocker dans le réservoir de liquide (134),
un conduit de liquide (156) qui est adapté pour recevoir du liquide (172) à partir de la soupape d'accueil (146) et qui s'ouvre dans le réservoir de liquide rechargeable (134) à une distance prédéterminée (A) de la soupape d'accueil (146), et
une soupape (181) pour libérer du gaz à partir du réservoir de liquide rechargeable (134) lors du rechargement du réservoir de liquide rechargeable (134), la soupape (181) comprenant un élément d'étanchéité flexible (182) et un contre-élément d'étanchéité essentiellement rigide (186) formé par une section de paroi latérale extérieure (183) du passage central (132).

2. Dispositif de cigarette électronique (100) selon la revendication 1, dans lequel le conduit de liquide (156) s'ouvre dans le réservoir de liquide rechargeable (134) avec un orifice de sortie (166).

3. Dispositif de cigarette électronique (100) selon la revendication 2, dans lequel l'orifice de sortie (166) est agencé plus près d'une section de paroi latérale (170) du réservoir de liquide rechargeable (134) opposée à la soupape d'accueil (146) que la soupape d'accueil (146).

4. Dispositif de cigarette électronique (100) selon la revendication 1, dans lequel la section du passage central (132) qui fournit le contre-élément d'étanchéité rigide (186) a une forme incurvée.

5. Dispositif de cigarette électronique (100) selon la revendication 1 ou 4, dans lequel le conduit de liquide (156) s'étend essentiellement perpendiculairement à et à travers le passage central (132) au niveau d'une section convexe (176) du passage central (132).

6. Dispositif de cigarette électronique (100) selon l'une quelconque des revendications 1 à 5, dans lequel le dispositif de cigarette électronique (100) comprend un couvercle d'embout (140) en communication avec un port d'inhalation d'air (136) pour fournir du liquide atomisé à un utilisateur, le couvercle d'embout (140) comprenant une position de rechargement (R) et une position de vapotage (V), dans la position de vapotage (V), le couvercle d'embout (140) étant plus proche d'une extrémité opposée du dispositif de cigarette électronique (100) que dans la position de rechargement (R), et/ou dans la position de vapotage (V), le couvercle d'embout (140) étant tourné autour d'un axe longitudinal du dispositif de cigarette électronique (100) d'un angle prédéterminé en comparaison de la position de rechargement (R).

7. Dispositif de cigarette électronique (100) selon la revendication 6, dans lequel la soupape d'accueil (146) est couverte par le couvercle d'embout (140) dans la position de vapotage (V), et est accessible lorsque le couvercle d'embout (140) est dans la position de rechargement (R).

8. Dispositif de cigarette électronique (100) selon la revendication 6 ou 7, dans lequel le couvercle d'embout (140) comprend un élément de fermeture (184) qui force l'élément d'étanchéité flexible (182) contre le contre-élément d'étanchéité rigide (186) lorsque le couvercle d'embout (140) est dans la position de vapotage (V).

9. Dispositif de cigarette électronique (100) selon la revendication 8, dans lequel l'élément de fermeture (184) est agencé à une distance de l'élément d'étanchéité flexible (182) lorsque le couvercle d'embout (140) est dans la position de rechargement (R).

10. Dispositif de cigarette électronique (100) selon l'une quelconque des revendications 6 à 9, dans lequel le couvercle d'embout (140) est maintenu dans la position de rechargement (R) et/ou dans la position de vapotage (V) par accouplement de force et/ou par accouplement de forme.

11. Dispositif de cigarette électronique (100) selon l'une quelconque des revendications 8 à 9, dans lequel un élément de verrou (188) d'une connexion à verrou maintenant le couvercle d'embout (140) dans la position de vapotage (V) et/ou dans la position de rechargement (R) est fourni par l'élément de fermeture (184), et un contre-élément de verrou (190, 192) est fourni par une section de paroi latérale extérieure (194) du passage central (132).

12. Dispositif de cigarette électronique (100) selon l'une quelconque des revendications 1 à 11, dans lequel la soupape d'accueil (146) est adaptée pour former une connexion encliquetable avec une contre-soupape d'accueil (150) d'une source de liquide.

13. Dispositif de cigarette électronique (100) selon l'une quelconque des revendications 1 à 12, dans lequel la soupape d'accueil (146) a une section d'accueil, la section d'accueil comprenant une forme convexe ou concave.

14. Portion d'atomiseur/réservoir de liquide (114) comprenant :
un réservoir de liquide (134),
un atomiseur (26) utilisable lorsqu'il est connecté à une alimentation électrique (18) pour un dispositif de cigarette électronique (100) pour atomiser du liquide stocké dans le réservoir de liquide (134), et
un passage central (132) qui est adapté pour conduire du liquide atomisé, le passage central (132) s'étendant à travers le réservoir de liquide rechargeable (134),
la portion d'atomiseur/réservoir de liquide (114) comprenant
une soupape d'accueil (146) pour accueillir une source de liquide (148) à stocker dans le réservoir de liquide (134),
un conduit de liquide (156) qui est adapté pour recevoir du liquide (172) à partir de la soupape d'accueil (146) et qui s'ouvre dans le réservoir de liquide rechargeable (134) à une distance prédéterminée (A) de la soupape d'accueil (146), et
une soupape (181) pour libérer du gaz à partir du réservoir de liquide rechargeable (134) lors du rechargement du réservoir de liquide rechargeable (134), la soupape (181) comprenant un élément d'étanchéité flexible (182) et un contre-élément d'étanchéité essentiellement rigide (186) formé par une section de paroi latérale extérieure (183) du passage central (132).
